# EUROPEAN PATENT SPECIFICATION

(11) **EP 4 572 767 B1**
(45) Date of publication and mention of the grant of the patent: **06.05.2026**
(21) Application number: 22955857.2
(22) Date of filing: 16.08.2022
(51) Int. Cl.: A61K 31/585, A61K 31/167, A61P 31/14, A61K 31/609

(54) **COMBINATION OF NICLOSAMIDE AND SPIRONOLACTONE FOR USE IN THE TREATMENT OF FCOV INFECTION OR FELINE INFECTIOUS PERITONITIS**
KOMBINATION VON NICLOSAMID UND SPIRONOLACTON ZUR VERWENDUNG BEI DER BEHANDLUNG VON FCOV-INFEKTIONEN ODER INFEKTIÖSER PERITONITIS BEI KATZEN
COMBINAISON DE NICLOSAMIDE ET DE SPIRONOLACTONE POUR LE TRAITEMENT DE L'INFECTION PAR LE VIRUS FCOV OU DE LA PÉRITONITE INFECTIEUSE FÉLINE

(43) Date of publication of application: 25.06.2025
(73) Proprietor: Imuneks Farma Ilac San. Ve Tic. A.S., 34349 Besiktas/Istanbul (TR)
(72) Inventor: PISAK, Mehmet Nevzat, 34337 Besiktas/Istanbul (TR)
(74) Representative: Yigiter, Zekiye Basak
(86) International application number: PCT/TR2022/050866
(87) International publication number: WO 2024/039311

(56) References cited:
- US-A1- 2020 016 155
- US-A1- 2020 016 155
- TANAKA YOSHIKAZU ET AL: "Ionophore Antibiotics Inhibit Type II Feline Coronavirus Proliferation In Vitro", VIRUSES, vol. 14, no. 8, 6 August 2022 (2022-08-06), CH, pages 1 - 13, XP093145356, ISSN: 1999-4915, DOI: 10.3390/v14081734
- YANG CHENG-WEI ET AL: "Repurposing old drugs as antiviral agents for coronaviruses", BIOMEDICAL JOURNAL, vol. 43, no. 4, 1 August 2020 (2020-08-01), NL, pages 368 - 374, XP055854406, ISSN: 2319-4170, DOI: 10.1016/j.bj.2020.05.003
- ERSOY AYSIN ET AL: "Covid19 ARDS olgularinda spironolaktonun etkinliginin degerlendirilmesi Assessment Of The Efficacy Of Spironolactone For COVID-19 ARDS Patients", AYDIN SAGLIK DERGISI, vol. 7, no. 3, 1 January 2015 (2015-01-01), pages 191 - 209, XP093145362, ISSN: 2149-5769, DOI: 10.17932/IAU.ASD.2015.007/asd_v07i3002
- TANAKA YOSHIKAZU, TANABE ERI, NONAKA YUKI, UEMURA MITSUKI, TAJIMA TSUYOSHI, OCHIAI KAZUHIKO: "Ionophore Antibiotics Inhibit Type II Feline Coronavirus Proliferation In Vitro", VIRUSES, vol. 14, no. 8, CH , pages 1 - 13, XP093145356, ISSN: 1999-4915, DOI: 10.3390/v14081734
- YANG, C. W. ET AL.: "Repurposing old drugs as antiviral agents for coronaviruses", BIOMEDICAL JOURNAL, vol. 43, no. 4, 2020, pages 368 - 374, XP055854406, DOI: 10.1016/j.bj.2020.05.003
- ERSOY AYSIN, GÜVEN BÜLENT BARIŞ, ERTÜRK TUNA, YURTSEVEN FULYA, KARAMAN ZÖHRE, GÜNER TEMEL, KÖMPE ÖZGE: "Covid19 ARDS olgularında spironolaktonun etkinliğinin değerlendirilmesi Assessment Of The Efficacy Of Spironolactone For COVID-19 ARDS Patients", AYDIN SAĞLIK DERGİSİ, vol. 7, no. 3, 1 January 2015 (2015-01-01), pages 191 - 209, XP093145362, ISSN: 2149-5769, DOI: 10.17932/IAU.ASD.2015.007/asd_v07i3002

## Description

### TECHNICAL FIELD

The present invention provides a pharmaceutical composition comprising niclosamide and spironolactone for the treatment of FCoV infection or feline infectious peritonitis.

### BACKGROUND ART

Animal epidemic diseases are the primary threat to the livestock and companion (pet) animals. Of the veterinary pathogens that cause these diseases, viruses are responsible for approximately half of the most important animal diseases. Animal viruses are divided into DNA and RNA viruses based on the genetic materials. The differences between DNA and RNA viruses include their lifetimes in target cells, how they attach to and enter host cells, and their biosynthesis, maturation, and release from cells.

Compared with DNA viruses, RNA viruses have a higher mutation rate and cause more serious damage in the livestock industry. Lower mutation rates of DNA viruses were usually influenced by the viral genome and DNA repairing protein that benefit for proofread and correct replication errors. Oppositely, offspring of RNA viruses are usually produced 1 ± 2 mutations compared with their parent. RNA viruses are also divided into avian, fish, mammalian, and zoonotic viruses in domestic animal industries.

There are more RNA viruses also being reported, apart from the classical swine fever virus (CSFV) and porcine reproductive and respiratory syndrome virus (PRRSV) which are the main RNA viruses that cause great losses in porcine industries. Because of their high mutation rates and the additional subgroups identified in recent years, both viruses are difficult to eliminate. Some RNA viruses not only infect animals but also humans, with the influenza virus the most typical RNA zoonotic virus. For example, H5N1 and H9N2 subgroups of the influenza virus are the main infectious pathogens affecting both humans and animals. Additionally, some RNA viruses primarily affecting humans, their transmission is directly related to contact with the virus containing body excretions of infected animals. For example, the main infection source of human Ebolavirus is close contact with pigs, dogs and non human primates. Similarly, Crimean-Congo haemorrhagic fever caused by *Nairoviruses,* may cause endemic outbreaks via transmission of the viruses from subclinically infected farm animals.

Feline infectious peritonitis (FIP) is a common disease and a frequent reason for mortality; approximately 1 of every 200 new feline cases presented to American veterinary teaching hospitals represents a cat with FIP. It is also a major factor in kitten mortality. FIP is a fatal immune-mediated disease triggered by the specific genetic mutations that is giving monocyte invasion ability to the virus in feline coronavirus (FCoV) infected cats. FCoV belongs to the family *Coronaviridae,* a group of enveloped positive-stranded RNA viruses that are frequently found in cats.

FCoV is distributed worldwide in household and stray cats. The virus is endemic especially in environments in which many cats are kept together in a small space (e.g., catteries, shelters, pet stores). There is virtually no multiple-cat household without endemic FCoV. Thus, once a cat in a household has contracted the disease, it is also important to provide prophylaxis for the healthy cats.

There are two main forms of FIP: effusive (wet) and non-effusive (dry). While both types are fatal, the effusive form is more common (60-70% of all cases) and progresses more rapidly than the non-effusive form. In both forms of the disease, the mortality is 90-95% once the symptoms begin to manifest.

There is a search for an effective antiviral treatment for cats with FIP. But unfortunately, most have not been very successful, although some studies have been performed with several antiviral therapies such as interferon and ribavirin. The only treatment with success so far has been the nucleoside analog GS-441524 which has 84-day treatment duration by injection and is only sold as a black-market product, since it is not currently approved by any medical authorities for human or veterinary diseases (Tasker et al, Viruses. 2021 Nov; 13(11):2228).

Niclosamide is an anthelmintic drug that has been approved for human and animal use for over 50 years. It has been found to possess inhibitory effects against the SARS-CoV-2 virus (see Yoshikazu et al., Viruses, 2022, 14 (8), 1-13).

In addition, spironolactone is an aldosterone antagonist that is used as a potassium sparing diuretic in dogs or cats that develop low potassium on other diuretic medications. It can be used as adjunctive treatment in congestive heart failure or as an adjunctive treatment for hypertension at a dose of 1-2 milligrams per kilogram orally every 12 hours for cats and at a dose of 0.5 to 4 milligrams per kilogram orally every 12-24 hours for dogs.

However, there is still a need in the art for the treatment of RNA virus diseases, especially feline infectious peritonitis in animals such as cats and for a pharmaceutical compound or combination of compounds capable of efficiently treating these diseases.

### SUMMARY OF THE INVENTION

The invention is set out in the appended set of claims.

One aspect of the present invention is to provide a combination comprising niclosamide and spironolactone for use in the treatment of FCoV infection or feline infectious peritonitis in a subject in need thereof, preferably for use in the treatment of FCoV infection and feline infectious peritonitis in cats.

### DETAILED DESCRIPTION OF THE INVENTION

The present invention provides a combination comprising niclosamide and spironolactone for use in the treatment of FCoV infection or feline infectious peritonitis in a subject in need thereof.

The present invention further relates to a combination comprising niclosamide and spironolactone for simultaneous, separate, or sequential use in the treatment described herein.

According to the present invention, niclosamide and spironolactone is preferably administered as an immediate release formulation.

According to the present invention, niclosamide is administered to the subject within a range of 10 to 300 mg per unit dose or 5 to 80 mg/kg per the weight of the subject. Preferably niclosamide is administered to the subject within a range of 20 to 200 mg per unit dose or 8 to 40 mg/kg per the weight of the subject. Most preferably; niclosamide is administered to the subject within a range of 30 to 150 mg per unit dose or 10 to 30 mg/kg per the weight of the subject. The inventors have surprisingly found that the low dose of niclosamide is enough to treat the diseases according to the present invention when used combined with spironolactone.

According to the present invention, spironolactone is administered to the subject within a range of 0.1 to 25 mg per unit dose or 0.3 to 3 mg/kg per the weight of the subject.

Preferably spironolactone is administered to the subject within a range of 0.5 to 20 mg per unit dose or 0.4 to 2 mg/kg per the weight of the subject. Most preferably; spironolactone is administered to the subject within a range of 1 to 10 mg per unit dose or 0.5 to 1.6 mg/kg per the weight of the subject.

The combination according to the present invention is administered 3 to 4 times in a day.

The subject treated according to the present invention may be human or animal, preferably animal; more preferably mammal, and most preferably cat.

In one embodiment of the present invention, the combination of niclosamide and spironolactone for use in the treatment of FCoV infection or feline infectious peritonitis in a subject in need thereof, may be in a fixed pharmaceutical composition or separate pharmaceutical compositions; preferably fixed pharmaceutical composition.

Niclosamide and spironolactone may be administered orally, subcutaneously, intravenously, or rectally and preferably orally. The pharmaceutical composition of niclosamide or spironolactone may be selected from the group consisting of tablet, effervescent tablet, capsule, powder or liquid, pellet, granule, solution, suspension, or syrup.

In another embodiment, the present invention provides a pharmaceutical composition comprising niclosamide, spironolactone and at least one pharmaceutically acceptable excipient. Accordingly, the pharmaceutical composition may be used in the treatment of FCoV infection or feline infectious peritonitis in a subject in need thereof; preferably for use in the treatment of feline infectious peritonitis in cats.

In one embodiment of the present invention, the combination of niclosamide and spironolactone may further be combined with another active ingredient for use in the treatment FCoV infection or feline infectious peritonitis in a subject in need thereof. Animals infected with RNA viruses and FCOV in particular also develop secondary conditions. These conditions may be treated with other active ingredients.

According to the present invention, the combination of niclosamide and spironolactone may be administered by mixing into the animal food and drinking water, when used in the treatment of animals suffering from conditions caused by FCoV infection or feline infectious peritonitis.

### Examples:

### Study to test the efficacy of a combination containing spironolactone and niclosamide

The study consisted of 20 cats of both sexes with clinical signs of FIP disease. The research was carried out by obtaining the ethics committee approval and informed consent from the cat owners included in the study. The treatment protocol described below was applied within the framework of a clinically controlled treatment plan. Accordingly, cats manifesting clinical FIP symptoms were evaluated as FIP positive according to clinical, serological, biochemical examination and PCR test results.

### Inclusion Criteria

Clinical symptoms persist for a maximum of 3 days
Weight over 1.5 kg
Fluid volume in body cavities below 100 ml in cases of wet FIP
ELISA positive
Albumin/Globulin ratio < 0.8

### Exclusion Criteria:

Organ failures that affect quality of life, incoordination and painful granulomatous lesions that affect vital functions.

Conditions that require treatment and medication other than the treatment protocol.

Administration of vaccines, immunostimulants and other antiviral drugs and biological agents.

### Diagnostic Protocol:

The diagnostic protocol for inclusion, described in detail below was followed for cats who applied to the clinic with clinical suspicion of FIP.

Accordingly; 18 g of blood was drawn from V. cephalica antebrachii with a needle and transferred to serum tubes for FCoV-ELISA (Immunocomb FCoV ELISA Test Kit, Biogal Inc) and biochemistry tests, and put into EDTA tubes for PCR and complete blood count analyses. Afterwards, the serum antibody level was determined by analyzing the immunocomb FCoV according to the ELISA test protocol (http://www.biogal.com/wp-content/uploads/2019/07/63FFP1021-1.pdf). It was frozen at -20 °C until PCR analysis was performed, and then RNA isolation was performed, and complementary DNA analysis was performed. Afterwards, Reverse Transciptase PCR analysis was performed using FCoV and FeLV specific primers. Complete blood counts were performed on the Mindray BC5000 hemogram device, and biochemical analyzes (ALT, AST, Albumin, globulin, urea, creatinine) were performed on the IDEXX Catalyst One Chemical Analyzer. In all cats included in the study, the presence of effusion was checked by thorax and abdomen x-rays and ultrasound examination.

### Treatment Protocol:

The following treatment was applied to all patients. Cats with lethargic conditions were hospitalized during the treatment and intravenous access was established and 20% Dextrose and 0.9% isotonic serum and supportive fluid were administered.

Niclosamide was mixed with the appropriate dose of spironolactone as detailed below:
The experimental fixed dose combination was obtained by mixing a 100 ml volume of 20mg/1 ml niclosamide suspension with 66 mg of spironolactone. The suspension is a conventional formulation obtained by conventional methods known in the prior art.

Which resulted in a formulation containing 20 mg of niclosamide and 0.66 mg of spironolactone per 1 ml.

| **Active ingredients** | **Dosage and administration** | **Dosing Schedule** |
|---|---|---|
| Niclosamide 20 mg/ml + spironolactone 0.66 mg/ml Oral Suspension | 1 ml/kg oral administration | Every 8 hours for 1 month |

### Study Results:

Four (4/8; 50%) of wet FIP patients who were treated died between the 4th and 17th days of treatment. In the necropsy-pathological examination of one of the dead cats; Necrotic and interstitial pneumonia was diagnosed and *T. gondii* infection was diagnosed antigenically. The 4 surviving cats were discharged after the completion of the treatment at the end of 4 weeks and after clinical and laboratory examinations. In terms of 8-month survival after treatment, it was noted that all cats survived (4/4, 100%). However, clinical improvement was observed in all treated dry FIP cases, with a 4-week survival rate (12/12, 100%). Of these cats, 2 died at 3 and 5 months after treatment was completed. However, the cause of death could not be clarified since autopsy could not be performed.

As a result; Since 4 of the 20 cats treated in the study died before the treatment was completed, 2 died after the treatment; The success rate of the FIP treatment protocol applied was the success rate of 14 out of 20 cats with FIP, and the efficacy rate was calculated according to the number of cats who were healthy 8 months post treatment, without any symptoms of viral infection; which was on average 14/20 (70%). The success rate was (4/8, 50%) for wet FIP cases, while the success rate for dry FIP cases was (10/12, 83.3%).

## Claims

1. A combination of niclosamide and spironolactone for use in the treatment of FCoV infection or feline infectious peritonitis.

2. A combination for use according to claim 1, wherein niclosamide and spironolactone are administered simultaneously, sequentially or separately.

3. A combination for use according to any one of the preceding claims, wherein niclosamide and spironolactone are administered simultaneously.

4. A combination for use according to any one of the preceding claims, wherein niclosamide and spironolactone are administered as immediate release formulation.

5. A combination for use according to any one of the preceding claims, wherein niclosamide is administered to the subject within a range of 5 to 80 mg/kg per the weight of the subject.

6. A combination for use according to claim 5, wherein niclosamide is administered to the subject within a range of 8 to 40 mg/kg per the weight of the subject.

7. A combination for use according to any one of the preceding claims, wherein niclosamide is administered to the subject within a range of 10 to 300 mg per unit dose.

8. A combination for use according to claim 7, wherein niclosamide is administered to the subject within a range of 20 to 200 mg per unit dose.

9. A combination for use according to any one of the preceding claims, wherein spironolactone is administered to the subject within a range of 0.3 to 3 mg/kg per the weight of the subject.

10. A combination for use according to claim 9, wherein spironolactone is administered to the subject within a range of 0.4 to 2 mg/kg per the weight of the subject.

11. A combination for use according to claim 10, wherein spironolactone is administered to the subject within a range of 0.5 to 1.6 mg/kg per the weight of the subject.

12. A combination for use according to any one of the preceding claims, wherein spironolactone is administered to the subject within a range of 0.1 to 25 mg/kg per unit dose.

13. A combination for use according to claim 12, wherein spironolactone is administered to the subject within a range of 0.5 to 20 mg/kg per unit dose.

14. A combination for use according to any one of the preceding claims, wherein niclosamide and spironolactone are administered 3 or 4 times in a day.

15. A combination for use according to any one of the preceding claims, wherein niclosamide and spironolactone are administered in a fixed pharmaceutical composition.

## Patentansprüche

1. Kombination aus Niclosamid und Spironolacton zur Verwendung bei der Behandlung von FCoV-Infektionen oder infektiöser Peritonitis bei Katzen.

2. Kombination zur Verwendung nach Anspruch 1, wobei Niclosamid und Spironolacton gleichzeitig, nacheinander oder getrennt verabreicht werden.

3. Kombination zur Verwendung nach irgendeinem der vorstehenden Ansprüche, wobei Niclosamid und Spironolacton gleichzeitig verabreicht werden.

4. Kombination zur Verwendung nach irgendeinem der vorstehenden Ansprüche, wobei Niclosamid und Spironolacton als sofort freisetzende Formulierung verabreicht werden.

5. Kombination zur Verwendung nach irgendeinem der vorstehenden Ansprüche, wobei Niclosamid dem Subjekt in einer Dosierung von 5 bis 80 mg/kg Körpergewicht des Subjekts verabreicht wird.

6. Kombination zur Verwendung nach Anspruch 5, wobei Niclosamid dem Subjekt in einer Dosierung zwischen 8 und 40 mg/kg Körpergewicht des Subjekts verabreicht wird.

7. Kombination zur Verwendung nach irgendeinem der vorstehenden Ansprüche, wobei Niclosamid dem Subjekt in einer Dosierung zwischen 10 und 300 mg pro Einzeldosis verabreicht wird.

8. Kombination zur Verwendung nach Anspruch 7, wobei Niclosamid dem Subjekt in einer Dosierung zwischen 20 und 200 mg pro Einzeldosis verabreicht wird.

9. Kombination zur Verwendung nach einem der vorstehenden Ansprüche, wobei Spironolacton dem Subjekt in einer Dosierung zwischen 0,3 und 3 mg/kg Körpergewicht des Subjekts verabreicht wird.

10. Kombination zur Verwendung nach Anspruch 9, wobei Spironolacton dem Subjekt in einer Dosierung von 0,4 bis 2 mg/kg Körpergewicht des Subjekts verabreicht wird.

11. Kombination zur Verwendung nach Anspruch 10, wobei Spironolacton dem Subjekt in einer Dosierung zwischen 0,5 und 1,6 mg/kg Körpergewicht des Subjekts verabreicht wird.

12. Kombination zur Verwendung nach irgendeinem der vorstehenden Ansprüche, wobei Spironolacton dem Subjekt in einer Dosierung zwischen 0,1 und 25 mg/kg pro Einzeldosis verabreicht wird.

13. Kombination zur Verwendung nach Anspruch 12, wobei Spironolacton dem Subjekt in einer Dosierung zwischen 0,5 und 20 mg/kg pro Einzeldosis verabreicht wird.

14. Kombination zur Verwendung nach irgendeinem der vorstehenden Ansprüche, wobei Niclosamid und Spironolacton 3- oder 4-mal täglich verabreicht werden.

15. Kombination zur Verwendung nach irgendeinem der vorstehenden Ansprüche, wobei Niclosamid und Spironolacton in einer festen pharmazeutischen Zusammensetzung verabreicht werden.

## Revendications

1. Une combinaison de niclosamide et de spironolactone à utiliser dans le traitement de l'infection par FCoV ou de la péritonite infectieuse féline.

2. Une combinaison à utiliser selon la revendication 1, dans laquelle le niclosamide et la spironolactone sont administrés simultanément, séquentiellement ou séparément.

3. Une combinaison à utiliser selon l'une quelconque des revendications précédentes, dans laquelle le niclosamide et la spironolactone sont administrés simultanément.

4. Une combinaison à utiliser selon l'une quelconque des revendications précédentes, dans laquelle le niclosamide et la spironolactone sont administrés sous forme de formulation à libération immédiate.

5. Une combinaison à utiliser selon l'une quelconque des revendications précédentes, dans laquelle le niclosamide est administré au sujet dans une plage de 5 à 80 mg/kg par rapport au poids du sujet.

6. Une combinaison à utiliser selon la revendication 5, dans laquelle le niclosamide est administré au sujet dans une plage de 8 à 40 mg/kg par rapport au poids du sujet.

7. Une combinaison à utiliser selon l'une quelconque des revendications précédentes, dans laquelle le niclosamide est administré au sujet dans une plage de 10 à 300 mg par dose unitaire.

8. Une combinaison à utiliser selon la revendication 7, dans laquelle le niclosamide est administré au sujet dans une plage de 20 à 200 mg par dose unitaire.

9. Une combinaison à utiliser selon l'une quelconque des revendications précédentes, dans laquelle la spironolactone est administrée au sujet dans une plage de 0,3 à 3 mg/kg par rapport au poids du sujet.

10. Une combinaison à utiliser selon la revendication 9, dans laquelle la spironolactone est administrée au sujet dans une plage de 0,4 à 2 mg/kg par rapport au poids du sujet.

11. Une combinaison à utiliser selon la revendication 10, dans laquelle la spironolactone est administrée au sujet dans une plage de 0,5 à 1,6 mg/kg par rapport au poids du sujet.

12. Une combinaison à utiliser selon l'une quelconque des revendications précédentes, dans laquelle la spironolactone est administrée au sujet dans une plage de 0,1 à 25 mg/kg par dose unitaire.

13. Une combinaison à utiliser selon la revendication 12, dans laquelle la spironolactone est administrée au sujet dans une plage de 0,5 à 20 mg/kg par dose unitaire.

14. Une combinaison à utiliser selon l'une quelconque des revendications précédentes, dans laquelle le niclosamide et la spironolactone sont administrés 3 ou 4 fois par jour.

15. Une combinaison à utiliser selon l'une quelconque des revendications précédentes, dans laquelle le niclosamide et la spironolactone sont administrés dans une composition pharmaceutique fixe.
